# EUROPÄISCHE PATENTSCHRIFT

(11) **EP 1 735 093 B1**
(45) Veröffentlichungstag und Bekanntmachung des Hinweises auf die Patenterteilung: **01.08.2012**
(21) Anmeldenummer: 05728204.8
(22) Anmeldetag: 24.03.2005
(51) Int. Cl.: B01J 23/68, B01J 21/06, B01J 37/02, B01J 37/04, C07C 51/265

(54) **KATALYSATOR MIT EINER SILBER-VANADIUMOXIDPHASE UND EINER PROMOTORPHASE**
CATALYST HAVING A SILVER-VANADIUM OXIDE PHASE AND A PROMOTER PHASE
CATALYSEUR PRESENTANT UNE PHASE D'OXYDE ARGENT-VANADIUM ET UNE PHASE PROMOTEUR

(30) Priorität: 26.03.2004 DE 102004014918
(43) Veröffentlichungstag der Anmeldung: 27.12.2006
(73) Patentinhaber: BASF SE, 67056 Ludwigshafen (DE)
(72) Erfinder: NETO, Samuel, 68167 Mannheim (DE); ROSOWSKI, Frank, 68165 Mannheim (DE); STORCK, Sebastian, 68167 Mannheim (DE); BAUER, Stefan, 67069 Ludwigshafen (DE)
(86) Internationale Anmeldenummer: PCT/EP2005/003179
(87) Internationale Veröffentlichungsnummer: WO 2005/092496

(56) Entgegenhaltungen:
- EP-A- 0 447 267
- WO-A-00/27753
- WO-A-01/85337
- WO-A-99/62637
- WO-A-2005/012216

## Beschreibung

Die Erfindung betrifft einen Katalysator mit einer Silber-Vanadiumoxidphase und einer Promotorphase und ein Verfahren zur Herstellung von Aldehyden, Carbonsäuren und/oder Carbonsäureanhydriden unter Verwendung des Katalysators.

Bekanntermaßen wird eine Vielzahl von Aldehyden, Carbonsäuren und/oder Carbonsäureanhydriden technisch durch die katalytische Gasphasenoxidation von aromatischen Kohlenwasserstoffen wie Benzol, o-, m- oder p-Xylol, Naphthalin, Toluol oder Durol (1,2,4,5-Tetramethylbenzol) in Festbettreaktoren, vorzugsweise Rohrbündelreaktoren, hergestellt. Dabei werden je nach Ausgangsmaterial beispielsweise Benzaldehyd, Benzoesäure, Maleinsäureanhydrid, Phthalsäureanhydrid, Isophthalsäure, Terephthalsäure oder Pyromellithsäureanhydrid gewonnen. Dazu wird im Allgemeinen ein Gemisch aus einem molekularen Sauerstoff enthaltenden Gas, beispielsweise Luft und das zu oxidierende Ausgangsmaterial durch Rohre geleitet, in denen sich eine Schüttung eines Katalysators befindet.

Die EP-A 447 267 beschreibt einen Katalysator zur Herstellung von Phthalsäureanhydrid durch Gasphasenoxidation von o-Xylol und/oder Naphthalin, der eine auf einem hitzefesten anorganischen Träger geträgerte Aktivmasse aufweist, die (A) 1 bis 20 Gew.-Teile V₂O₅ und 99 bis 80 Gew.-Teile Anatas und (B) auf 100 Gew.-Teile (A) 0,05 Gew.-Teile Oxid von K, Cs, Rb und/oder Th und 0,05 bis 2 Gew.-Teile Ag₂O umfasst.

In der WO 00/27753, der WO 01/85337 und der älteren Patentanmeldung DE 10334132.3 werden Silber- und Vanadiumoxid enthaltende Multimetalloxide und deren Verwendung für die partielle Oxidation von aromatischen Kohlenwasserstoffen beschrieben.

Der Erfindung liegt die Aufgabe zu Grunde, die mit diesen Katalysatoren erreichten Ausbeuten ohne Beeinträchtigung der Selektivitäten zu verbessern.

Die Aufgabe wird erfindungsgemäß gelöst durch einen Katalysator mit einer katalytisch aktiven Masse, die eine Phase A und eine Phase B in Form dreidimensional ausgedehnter, von ihrer lokalen Umgebung aufgrund ihrer von ihrer lokalen Umgebung verschiedenen chemischen Zusammensetzung abgegrenzter Bereiche enthält, wobei die Phase A eine Silber-Vanadiumoxid-Bronze und die Phase B eine Mischoxidphase auf Basis von Titandioxid und Vanadiumpentoxid ist.

Die erfindungsgemäßen Katalysatoren sind Schalenkatalysatoren, d. h. die katalytisch aktive Masse ist in Form wenigstens einer Schale auf einen inerten Träger aufgebracht.

In einer Ausführungsform sind die Phasen A und B relativ zueinander wie in einem Gemisch aus feinteiligem A und feinteiligem B verteilt. Die Korngrößen der beiden Phasen liegen vorzugsweise im Bereich von 0,1 bis 800 µm, insbesondere 0,5 bis 100 µm, besonders bevorzugt 1 bis 100 µm.

In einer anderen Ausführungsform sind die Phasen A und B relativ zueinander als konzentrische Schalen angeordnet. In der Regel bringt man hierzu zunächst die Phase A (bzw. einen Vorläufer dafür oder Quellen der elementaren Konstituenten davon) schalenförmig auf einen inerten Träger auf und überzieht den so beschichteten Träger dann mit der Phase B (bzw. einem Vorläufer dafür oder Quellen der elementaren Konstituenten davon). Obwohl es bevorzugt ist, dass die Phase B die Phase A vollständig bedeckt, ist auch eine teilweise Bedeckung mit der Phase B denkbar.

Das Gewichtsverhältnis der Phase A zur Phase B liegt im Allgemeinen im Bereich von 80:20 bis 98:2, vorzugsweise 85:15 bis 95:5.

Die Erfindung betrifft außerdem ein Verfahren zur Herstellung eines vorstehend definierten Katalysators, bei dem man ein Pulver, das die Phase A, einen Vorläufer dafür oder Quellen der elementaren Konstituenten davon umfasst, und ein Pulver, das die Phase B, einen Vorläufer dafür oder Quellen der elementaren Konstituenten davon umfasst, vermengt und auf einen inerten Träger aufbringt.

Die Erfindung betrifft außerdem ein Verfahren zur Herstellung eines vorstehend definierten Katalysators, bei dem man nacheinander (i) die Phase A, einen Vorläufer dafür oder Quellen der elementaren Konstituenten davon und (ii) die Phase B, einen Vorläufer dafür oder Quellen der elementaren Konstituenten davon auf einen inerten Träger aufbringt.

Die Erfindung betrifft außerdem ein Verfahren zur Herstellung von Aldehyden, Carbonsäuren und/oder Carbonsäureanhydriden, bei dem man einen gasförmigen Strom, der einen aromatischen Kohlenwasserstoff und ein molekularen Sauerstoff enthaltendes Gas umfasst, bei erhöhter Temperatur mit einem vorstehend definierten Katalysator in Kontakt bringt.

Silber-Vanadiumoxid-Bronzen und ihre Herstellung sind an sich bekannt, z. B. aus der WO 00/27753 und der WO 01/85337. Es werden darunter Silber-Vanadiumoxid-Verbindungen mit einem atomaren Ag : V-Verhältnis von weniger als 1 verstanden. Es handelt sich im Allgemeinen um halbleitende oder metallisch leitfähige, oxidische Festkörper, die bevorzugt in Schicht- oder Tunnelstrukturen kristallisieren, wobei das Vanadium im [V₂O₅]-Wirtsgitter teilweise reduziert zu V(IV) vorliegt. Die katalytisch aktiven Silber-Vanadiumoxid-Bronzen bilden sich oberhalb 200 °C, insbesondere bei Temperaturen von mehr als 300 °C, durch Zersetzung bestimmter Multimetalloxide.

Die Phase A weist vorzugsweise eine Zusammensetzung auf, die erhältlich ist durch Calzinieren eines Multimetalloxids der allgemeinen Formel I

Ag_{a-c}M¹_{c}V₂Od * e H₂O, I

worin
- a: einen Wert von 0,3 bis 1,9 hat,
- M¹: für wenigstens ein unter Alkali- und Erdalkalimetallen, Bi, Tl, Cu, Zn, Cd, Pb, Cr, Au, Al, Fe, Co, Ni, Mo, Nb, Ce, W, Mn, Ta, Pd, Pt, Ru und/oder Rh ist ausgewähltes Metall steht,
- c: einen Wert von 0 bis 0,5 hat, mit der Maßgabe, dass (a-c) ≥ 0,1 ist,
- d: eine Zahl, die sich durch die Wertigkeit und Häufigkeit der von Sauerstoff verschiedenen Elemente in der Formel I bestimmt, bedeutet und
- e: einen Wert von 0 bis 20, vorzugsweise 0 bis 5, hat.

Im Multimetalloxid der Formel I hat die Variable a vorzugsweise einen Wert von 0,5 bis 1,0 und besonders bevorzugt 0,6 bis 0,9, der Wert der Variablen b beträgt vorzugsweise 0 bis 0,1, und der Wert der Variablen c beträgt vorzugsweise 0,005 bis 0,2, insbesondere 0,01 bis 0,1.

Die Zahl d bestimmt sich aus der Wertigkeit und Häufigkeit der von Sauerstoff verschiedenen Elemente im Multimetalloxid der Formel I. Die Zahl e, die ein Maß für den Wassergehalt ist, beträgt vorzugsweise 0 bis 5.

M¹ steht vorzugsweise für Na, K, Rb, Tl, Ni, W, Co, Fe, Mo, Nb, Zn, Ce und Mn.

Besonders bevorzugt sind Multimetalloxide der allgemeinen Formel la

AgₐV₂O_{d} * e H₂O, Ia

worin
- a: einen Wert von 0,6 bis 0,9 hat,

- d: die oben angegebene Bedeutung hat, und

- e: einen Wert von 0 bis 5 hat.

Die spezifische Oberfläche nach BET, gemessen gemäß DIN 66 131, die auf den "Recommendations 1984" der IUPAC International Union of Pure and Applied Chemistry (s. Pure & Appl. Chem. 57, 603 (1985)) basiert, beträgt in der Regel mehr als 1 m²/g, bevorzugt 3 bis 250 m²/g, insbesondere 10 bis 250 m²/g und besonders bevorzugt 20 bis 80 m²/g.

Vorzugsweise liegt das Multimetalloxid in einer Kristallstruktur vor, deren Pulverröntgendiagramm durch Beugungsreflexe bei den Netzebenenabständen d 15,23 ± 0,6, 12,16 ± 0,4, 10,68 ± 0,3, 3,41 ± 0,04, 3,09 ± 0,04, 3,02 ± 0,04, 2,36 ± 0,04 und 1,80 ± 0,04 Å gekennzeichnet ist. Die Angabe der Röntgenbeugungsreflexe erfolgt in dieser Anmeldung in Form der von der Wellenlänge der verwendeten Röntgenstrahlung unabhängigen Netzebenenabstände d[Å], die sich aus dem gemessenen Beugungswinkel mittels der Bragg'schen Gleichung errechnen lassen.

Zur Herstellung der Multimetalloxide wird im Allgemeinen eine Suspension von Vanadiumpentoxid (V₂O₅) mit der Lösung einer Silberverbindung sowie gegebenenfalls einer Lösung einer Verbindung der Metallkomponente M¹ erhitzt. Als Lösungsmittel für diese Umsetzung können polare organische Lösungsmittel, wie Polyole, Polyether oder Amine, z. B. Pyridin, dienen, bevorzugt wird als Lösungsmittel Wasser verwendet. Als Silbersalz wird bevorzugt Silbernitrat verwendet, die Verwendung anderer löslicher Silbersalze, z. B. Silberacetat, Silberperchlorat oder Silberfluorid ist ebenfalls möglich.

Als Salze der Metallkomponente M¹ werden in der Regel solche gewählt, die im verwendeten Lösungsmittel löslich sind. Wird Wasser als Lösungsmittel bei der Herstellung der erfindungsgemäßen Multimetalloxide verwendet, können beispielsweise die Perchlorate oder Carboxylate, insbesondere die Acetate, der Metallkomponente M¹ eingesetzt werden. Bevorzugt werden die Nitrate der betreffenden Metallkomponente M¹ verwendet.

Die Umsetzung des V₂O₅ mit der Silberverbindung und gegebenenfalls der Verbindung der Metallkomponente M¹ kann im Allgemeinen bei Raumtemperatur oder bei erhöhter Temperatur durchgeführt werden. In der Regel wird die Umsetzung bei Temperaturen von 20 bis 375 °C, vorzugsweise bei 20 bis 100 °C und besonders bevorzugt bei 60 bis 100 °C vorgenommen. Liegt die Temperatur der Umsetzung oberhalb der Temperatur des Siedepunktes des verwendeten Lösungsmittels, wird die Umsetzung zweckmäßigerweise unter dem Eigendruck des Reaktionssystems in einem Druckgefäß ausgeführt. Vorzugsweise werden die Reaktionsbedingungen so gewählt, dass die Umsetzung bei Atmosphärendruck durchgeführt werden kann. Die Dauer dieser Umsetzung kann in Abhängigkeit von der Art der umgesetzten Ausgangsmaterialien und den angewandten Temperaturbedingungen 10 Minuten bis 3 Tage betragen. Bei der Umsetzung verändert sich die orangerote Farbe der V₂O₅-Suspension und es bildet sich die neue Verbindung in Form einer dunkelbraunen Suspension.

Je nach der gewünschten chemischen Zusammensetzung des Multimetalloxids der Formel I werden zu dessen Herstellung die sich aus a und c von Formel I ergebenden Mengen von V₂O₅, Silberverbindung sowie der Verbindung der Metallkomponente M¹ miteinander umgesetzt. Nach beendeter Umsetzung wird dabei das Multimetalloxid mit faserförmiger Kristallmorphologie erhalten.

Das so gebildete Multimetalloxid kann aus der Reaktionsmischung isoliert und bis zur weiteren Verwendung gelagert werden. Die Isolierung des Multimetalloxids kann z. B. durch Abfiltrieren der Suspension und Trocknen des erhaltenen Feststoffs erfolgen, wobei die Trocknung sowohl in herkömmlichen Trocknern, aber auch z. B. in Gefriertrocknern durchgeführt werden kann. Besonders vorteilhaft wird die Trocknung der erhaltenen Multimetalloxid-Suspension mittels Sprühtrocknung durchgeführt. Es kann vorteilhaft sein, das bei der Umsetzung erhaltene Multimetalloxid vor dessen Trocknung salzfrei zu waschen. Die Sprühtrocknung wird im Allgemeinen unter Atmosphärendruck oder vermindertem Druck vorgenommen. Je nach angewandtem Druck und verwendetem Lösungsmittel bestimmt sich die Eingangstemperatur des Trocknungsgases - im Allgemeinen wird als solches Luft verwendet, es können aber selbstverständlich auch andere Trocknungsgase wie Stickstoff oder Argon, benutzt werden. Die Eingangstemperatur des Trocknungsgases in den Sprühtrockner wird vorteilhaft so gewählt, dass die Ausgangstemperatur des durch Verdampfung des Lösungsmittels abgekühlten Trocknungsgases 200 °C für einen längeren Zeitraum nicht übersteigt. In der Regel wird die Ausgangstemperatur des Trocknungsgases auf 50 bis 150 °C, vorzugsweise 100 bis 140 °C eingestellt.

Die Phase B enthält neben Titandioxid (in Form seiner Anatasmodifikation) Vanadiumpentoxid. Des weiteren können in geringen Mengen eine Vielzahl anderer oxidischer Verbindungen enthalten sein, die als Promotoren die Aktivität und Selektivität des Katalysators beeinflussen. Vorzugsweise enthält die Phase B im calzinierten Zustand 1 bis 20 Gew.-% Vanadiumoxid, berechnet als V₂O₅, und 80 bis 99 Gew.% Titandoxid, berechnet als TiO₂.

Als Promotoren seien beispielhaft die Alkalimetalloxide, wie Cäsiumoxid, Lithium-, Kalium- und Rubidiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Zinkoxid genannt. In der Regel wird aus dieser Gruppe Cäsium als Promotor verwendet.

Als die Aktivität erhöhenden aber die Selektivität vermindernden Zusatz kommen vor allem oxidische Phosphorverbindungen oder Ammoniumhydrogenphosphat in Betracht.

Vorzugsweise weist die Phase B eine Zusammensetzung der Formel II auf

VₓTi₁₋ₓM²_{y}M³_{w}O_{z} II

worin
- M²: für wenigstens ein Alkalimetall steht
- M³: für ein Element der 5. Hauptgruppe des Periodensystems der Elemente steht,
- x: einen Wert von 0,001 bis 0,2 hat,
- y: einen Wert von 0 bis 0,01 hat,
- w: einen Wert von 0 bis 0,02 hat, und
- z: eine Zahl, die sich durch die Wertigkeit und Häufigkeit der von Sauerstoff verschiedenen Elemente in der Formel II bestimmt, bedeutet.

M² steht vorzugsweise für Cs.

M³ steht vorzugsweise für Sb oder P.

Die Komponenten der Mischoxid-Phase B werden in Form deren Oxide oder in Form von Verbindungen eingesetzt, welche sich beim Erhitzen bzw. beim Erhitzen in Gegenwart von Sauerstoff in Oxide umwandeln. Als Vanadium-Komponente können Vanadiumoxide oder Vanadiumverbindungen, die sich beim Erhitzen in Vanadiumoxid umwandeln, einzeln oder in Form deren Gemische eingesetzt werden. Vorzugsweise werden V₂O₅ oder NH₄VO₃ eingesetzt. Man kann auch ein Reduktionsmittel, wie Ameisensäure oder Oxalsäure, mitverwenden, um die Vanadium(V)-Verbindung zumindest teilweise zu Vanadium(IV) zu reduzieren. Geeignete Alkalimetallverbindungen oder Verbindungen der Elemente der 5. Hauptgruppe des Periodensystems der Elemente sind die entsprechenden Oxide oder Verbindungen, die sich nach dem Erhitzen in Oxide umwandeln, wie Ammoniumsalze, Sulfate, Nitrate, Carbonate. Geeignet sind beispielsweise Na₂CO₃, K₂O, Cs₂O, Cs₂CO₃, Cs₂SO₄, P₂O₅, (NH₄)₂HPO₄, Sb₂O₃.

Zur Bildung der Phase B bereitet man im Allgemeinen eine wässrige Aufschlämmung der Verbindung der Vanadium-Komponente, des Titandioxids sowie von M² in geeigneten Mengen und rührt die Aufschlämmung, bis eine ausreichende Homogenisierung erreicht ist. Die Aufschlämmung kann dann sprühgetrocknet werden oder als solche zur Beschichtung verwendet werden.

Die Herstellung der erfindungsgemäßen Katalysatoren erfolgt zweckmäßigerweise über die Stufe eines so genannten "Präkatalysators", der als solcher gelagert und gehandelt werden kann und aus dem der aktive Katalysator entweder durch thermische Behandlung hergestellt oder in situ im Oxidationsreaktor unter den Bedingungen der Oxidationsreaktion erzeugt werden kann. Bei der thermischen Behandlung der Präkatalysatoren bei Temperaturen von über 200 bis 650 °C, vorzugsweise bei über 250 bis 500 °C, insbesondere bei 300 bis 450 °C, zersetzen sich die im Präkatalysator enthaltenen Multimetalloxide zu Silber-Vanadiumoxid-Bronzen, die die Phase A des erfindungsgemäßen Katalysators bilden. Diese Umwandlung der im Präkatalysator enthaltenen erfindungsgemäßen Multimetalloxide zu Silber-Vanadiumoxid-Bronzen findet insbesondere auch in situ im Reaktor zur Gasphasenpartialoxidation von aromatischen Kohlenwasserstoffen statt, wenn man den Präkatalysator bei dieser Umsetzung einsetzt. Die dabei entstehenden Silber-Vanadiumoxid-Bronzen sind katalytisch aktiver Bestandteil der katalytisch aktiven Schicht des erfindungsgemäßen Schalenkatalysators. Die thermische Umwandlung der Multimetalloxide zu Silber-Vanadiumoxid-Bronzen verläuft über eine Reihe von Reduktions- und Oxidationsreaktionen, die im Einzelnen noch nicht verstanden sind.

Als inertes nicht-poröses Trägermaterial für die erfindungsgemäßen Katalysatoren können praktisch alle Trägermaterialien des Standes der Technik, wie sie vorteilhaft bei der Herstellung von Schalenkatalysatoren für die Oxidation aromatischer Kohlenwasserstoffe zu Aldehyden, Carbonsäuren und/oder Carbonsäureanhydriden eingesetzt werden, Verwendung finden, beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Der Ausdruck "nicht-porös" ist dabei im Sinne von "bis auf technisch unwirksame Mengen an Poren nicht-porös" zu verstehen, da technisch unvermeidlich eine geringe Anzahl Poren im Trägermaterial, das idealerweise keine Poren enthalten sollte, vorhanden sein können. Als vorteilhafte Trägermaterialien sind insbesondere Steatit und Siliciumcarbid hervorzuheben. Die Form des Trägermaterials ist für die erfindungsgemäßen Präkatalysatoren und Schalenkatalysatoren im Allgemeinen nicht kritisch. Beispielsweise können Katalysatorträger in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenpartialoxidation von aromatischen Kohlenwasserstoffen verwendeten Katalysatorträgern.

Zur schalenförmigen Beschichtung des inerten Trägermaterials werden bekannte Verfahren angewendet. Beispielsweise kann eine Suspension der Aktivmasse oder eines Vorläufers in einer beheizten Dragiertrommel bei erhöhter Temperatur auf den Katalysatorträger aufgesprüht werden. Anstelle von Dragiertrommeln können auch Wirbelbettbeschichter eingesetzt werden.

Das Suspensionsmedium ist im Allgemeinen Wasser, dem vorzugsweise Bindemittel wie höhere Alkohole, mehrwertige Alkohole, z. B. Ethylenglykol, 1,4-Butandiol oder Glycerin, Dimethylformamid, Dimethylacetamid, Dimethylsulfoxid, N-Methylpyrrolidon oder cyclische Harnstoffe, wie N,N'-Dimethylethylenharnstoff oder N,N'-Dimethylpropylenharnstoff, oder (Co)Polymere, gelöst oder vorteilhaft in Form einer wässrigen Dispersion zugesetzt werden, wobei im Allgemeinen Bindemittelgehalte von 10 bis 20 Gew.-%, bezogen auf den Feststoffgehalt der Suspension geeignet sind. Geeignete polymere Bindemittel sind z. B. Vinylacetat/Vinyllaurat-, Vinylacetat/Acrylat-, Styroi/Acrylat-, Vinylacetat/Maleat- oder Vinylacetat/Ethylen-Copolymere. Bei einer thermischen Behandlung bei Temperaturen über 200 bis 500 °C entweicht das Bindemittel durch thermische Zersetzung und/oder Verbrennung aus der aufgetragenen Schicht.

Um Katalysatoren zu erhalten, in deren aktive Masse die Phasen A und B relativ zueinander wie in einem Gemisch aus feinteiligem A und feinteiligem B verteilt sind, vermengt man zweckmäßigerweise das nach Isolierung und Trocknung erhaltene Pulver des oben genannten Multimetalloxids mit einem Pulver, das die elementaren Konstituenten der Mischoxid-Phase B enthält, und bringt das Gemenge in der beschriebenen Weise auf den inerten Träger auf.

Die Schichtdicke der Katalysatorschale bzw. die Summe der Schichtdicken der Schalen, die die katalytisch aktiven Bestandteile enthalten, beträgt im Allgemeinen 10 bis 250 µm.

Die erfindungsgemäßen Katalysatoren werden für die partielle Oxidation von aromatischen oder heteroaromatischen Kohlenwasserstoffen zu Aldehyden, Carbonsäuren und/oder Carbonsäureanhydriden, insbesondere zur Gasphasenpartialoxidation von o-Xylol und/oder Naphthalin zu Phthalsäureanhydrid, von Toluol zu Benzoesäure und/oder Benzaldehyd, oder von Methylpyridinen, wie β-Picolin zu Pyridincarbonsäuren, wie Nicotinsäure, mit einem molekularen Sauerstoff enthaltenden Gas verwendet. Die erfindungsgemäßen Katalysatoren können zu diesem Zweck alleine oder in Kombination mit anderen, unterschiedlich aktiven Katalysatoren, beispielsweise Katalysatoren des Standes der Technik auf Vanadiumoxid/Anatas-Basis, eingesetzt werden, wobei die unterschiedlichen Katalysatoren im Allgemeinen in separaten Katalysatorschüttungen, die in einem oder mehreren Katalysatorfestbetten angeordnet sein können, im Reaktor angeordnet werden.

Die erfindungsgemäßen Schalenkatalysatoren oder Präkatalysatoren werden hierzu in die Reaktionsrohre eines Röhrenreaktors gefüllt, die von außen, z. B. mittels einer Salzschmelze, auf die Reaktionstemperatur thermostatisiert werden. Wird anstelle des erfindungsgemäßen Schalenkatalysators ein oben definierter Präkatalysator eingesetzt, entsteht daraus unter den Temperaturbedingungen der partiellen Oxidation ein erfindungsgemäßer Schalenkatalysator. Über die so bereitete Katalysatorschüttung wird das Reaktionsgas bei Temperaturen von 100 bis 650 °C und vorzugsweise 250 bis 480 °C und bei einem Überdruck von im Allgemeinen 0,1 bis 2,5 bar, vorzugsweise von 0,3 bis 1,5 bar mit einer Raumgeschwindigkeit von im Allgemeinen 750 bis 5000 h⁻¹ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel, wie Wasserdampf, Kohlendioxid und/oder Stickstoff, enthalten kann, mit dem zu oxidierenden, aromatischen Kohlenwasserstoff erzeugt, wobei das molekularen Sauerstoff enthaltende Gas im Allgemeinen 1 bis 100 Vol.-%, vorzugsweise 2 bis 50 Vol.% und besonders bevorzugt 10 bis 30 Vol.-% Sauerstoff, 0 bis 30 Vol.%, vorzugsweise 0 bis 20 Vol.% Wasserdampf sowie 0 bis 50 Vol.-%, vorzugsweise 0 bis 1 Vol.% Kohlendioxid, Rest Stickstoff, enthalten kann. Zur Erzeugung des Reaktionsgases wird das molekularen Sauerstoff enthaltende Gas im Allgemeinen mit 30 bis 300 g je Nm³, bevorzugt mit 70 bis 150 g je Nm³ Gas des zu oxidierenden aromatischen Kohlenwasserstoffs beschickt. Besonders vorteilhaft wird als molekularen Sauerstoff enthaltendes Gas Luft verwendet.

Vorteilhaft wird die Gasphasenpartialoxidation so durchgeführt, dass man zwei oder mehr Zonen, vorzugsweise zwei Zonen, der im Reaktionsrohr befindlichen Katalysatorschüttung auf unterschiedliche Reaktionstemperaturen thermostatisiert, wozu beispielsweise Reaktoren mit getrennten Salzbädern eingesetzt werden können. Wird die Umsetzung in zwei Reaktionszonen durchgeführt, wird im Allgemeinen die zum Gaseintritt des Reaktionsgases hin gelegene Reaktionszone, welche im Allgemeinen 30 bis 80 Vol.% des gesamten Katalysatorvolumens umfasst, auf eine um 1 bis 20 °C, vorzugsweise um 1 bis 10 °C und insbesondere um 2 bis 8 °C höhere Reaktionstemperatur als die zum Gasaustritt hin gelegene Reaktionszone thermostatisiert. Eine solche Arbeitsweise wird als Zwei- oder Mehrzonenstrukturierung des Reaktors bezeichnet. Alternativ kann die Gasphasenoxidation auch ohne Aufteilung in Temperaturzonen bei einer einheitlichen Reaktionstemperatur durchgeführt werden.

Bei einer bevorzugten Ausführungsform des Verfahrens zur partiellen Oxidation von aromatischen Kohlenwasserstoffen und Heterozyklen (z.B. Methylpyridin bzw. β-Picolin) zu Aldehyden, Carbonsäuren und/oder Carbonsäureanhydriden, die sich besonders vorteilhaft für die Herstellung von Phthalsäureanhydrid aus o-Xylol und/oder Naphthalin erweist, wird der aromatische Kohlenwasserstoff zunächst an einer Schüttung des erfindungsgemäßen Katalysators unter Teilumsatz zu einem Reaktionsgemisch umgesetzt. Das erhaltene Reaktionsgemisch oder eine Fraktion davon kann man dann mit wenigstens einem weiteren Katalysator in Kontakt bringen, dessen katalytisch aktive Masse Vanadiumpentoxid und Anatas enthält.

Im Fall der Herstellung von Phthalsäureanhydrid aus o-Xylol enthält das teilumgesetzte Reaktionsgemisch z. B. Phthalsäureanhydrid und andere Oxidationsprodukte, wie o-Tolylaldehyd, o-Tolylcarbonsäure und Phthalid, und nicht umgesetztes o-Xylol. Es kann dann weiterverarbeitet werden, indem entweder
a) das o-Xylol vom Phthalsäureanhydrid und den anderen Oxidationsprodukten, die Intermediate auf dem Reaktionsweg von o-Xylol zu Phthalsäureanhydrid sind, abgetrennt und zurückgeführt wird und der Strom aus Phthalsäureanhydrid und Intermediaten einer oder mehreren weiteren Katalysatorschüttungen mit z. B. einem Schalenkatalysator auf Vanadiumoxid/Anatas-Basis zugeführt wird, wo die Intermediate selektiv zu Phthalsäureanhydrid oxidiert werden; oder indem
b) das Produktgemisch ohne weitere Aufarbeitung, d. h. ohne o-Xylol-Abtrennung, über eine zweite oder gegebenenfalls über weitere Katalysatorschüttungen geleitet wird.

Vorzugsweise leitet man den gasförmigen Strom nacheinander über ein Bett eines stromaufwärts gelegenen Katalysators und ein Bett eines stromabwärts gelegenen Katalysators leitet, wobei das Bett des stromaufwärts gelegenen Katalysators einen erfindungsgemäßen Katalysator enthält und das Bett des stromabwärts gelegenen Katalysators wenigstens einen Katalysator enthält, dessen katalytisch aktive Masse Vanadiumpentoxid und Anatas enthält. Im Allgemeinen enthält die katalytisch aktive Masse des stromabwärts gelegenen Katalysators 1 bis 40 Gew.-% Vanadiumoxid, berechnet als V₂O₅, 60 bis 99 Gew.-% Titandioxid, berechnet als TiO₂, bis zu 1 Gew.-% einer Cäsiumverbindung, berechnet als Cs, bis zu 1 Gew.-% einer Phosphorverbindung, berechnet als P, und bis zu 10 Gew.-% Antimonoxid, berechnet als Sb₂O₃. Mit Vorteil umfasst das Bett des stromabwärts gelegenen Katalysators wenigstens zwei Lagen von Katalysatoren, deren katalytisch aktive Masse unterschiedlichen Cs-Gehalt aufweist, wobei der Cs-Gehalt im Strömungsrichtung des gasförmigen Stroms abnimmt.

Durch diese Art der Reaktionsführung wird insgesamt eine deutlich höhere Phthalsäureanhydrid-Ausbeute erzielt als bei alleiniger Verwendung von Katalysatorsystemen auf Vanadiumoxid/Anatas-Basis, da die erfindungsgemäßen Katalysatoren o-Xylol und/oder Naphthalin wesentlich selektiver zu Phthalsäureanhydrid bzw. den vorstehend genannten Intermediaten oxidieren können.

Auf analoge Weise kann bei der Oxidation von Toluol zu Benzaldehyd und/oder Benzoesäure beziehungsweise der Oxidation von β-Picolin zu Nicotinsäure verfahren werden. Benzaldehyd findet beispielsweise als Aromastoff Verwendung. Nicotinsäure findet beispielsweise als Ausgangsstoff für die Herstellung von Vitaminen Verwendung.

### Beispiele

### Katalysatoren

### A Herstellung des Multimetalloxids Ag_{0,73}V₂Oₓ

In 7 I vollentsalztes Wasser von 60 °C wurden 102 g V₂O₅ (= 0,56 Mol) unter Rühren zugegeben. In die erhaltene orangefarbene Suspension wurde unter weiterem Rühren eine wässrige Lösung von 69,5 g AgNO₃ (= 0,409 Mol) in 1 I Wasser zugegeben. Anschließend wurde die Temperatur der erhaltenen Suspension innerhalb von 2 Stunden auf 90 °C erhöht und bei dieser Temperatur die Mischung 24 Stunden gerührt. Danach wurde die erhaltene dunkelbraune Suspension abgekühlt und sprühgetrocknet (Eingangstemperatur (Luft) = 350 °C, Ausgangstemperatur (Luft) = 110 °C).

Das erhaltene Pulver hatte eine spezifische Oberfläche nach BET von 56 m²/g. Vom erhaltenen Pulver wurde ein Pulverröntgendiagramm mit Hilfe eines Diffraktometers D 5000 der Firma Siemens unter Anwendung von Cu-Kα-Strahlung (40 kV, 30 mA) aufgenommen. Das Diffraktometer war mit einem automatischen Primär- und Sekundärblendensystem sowie einem Sekundär-Monochromator und Szintillationsdetektor ausgestattet. Aus dem Pulverröntgendiagramm wurden die folgenden Netzebenenabstände d [Å] mit den dazugehörigen relativen Intensitäten Iᵣₑₗ [%] erhalten: 15,04 (11,9), 1 1,99 (8,5), 10,66 (15,1), 5,05 (12,5), 4,35 (23), 3,85 (16,9), 3,41 (62,6), 3,09 (55,1), 3,02 (100), 2,58 (23,8), 2,48 (27,7), 2,42 (25,1), 2,36 (34,2), 2,04 (26,4), 1,93 (33,2), 1,80 (35,1), 1,55 (37,8).

### B1 Herstellung einer V₂O₅/TiO₂-Phase mit 0,4 Gew.-% Cs

In 100 ml vollentsalztes Wasser von 60 °C gab man unter Rühren 13,0 g Oxalsäuredihydrat (= 0,12 Mol) und dann 3,35 g V₂O₅ (= 0,17 Mol). Die erhaltene blaue Lösung wurde 30 min unter weiterem Rühren auf 90 °C erhitzt. Nach dem Abkühlen auf Raumtemperatur fügte man 0,46 g Cäsiumsulfat (= 0,0013 Mol), 38,9 g Formamid, 80 g Anatas mit einer BET-Oberfläche von 21 m²/g und 157,5 g Wasser hinzu (Feststoffgehalt etwa 21 %). Anschließend rührte man die erhaltene Suspension 15 Stunden bei 25 °C. Dann wurde die Suspension sprühgetrocknet (Eingangstemperatur (Luft) = 350 °C; Ausgangstemperatur (Luft) = 110 °C). Das Sprühpulver bestand im Mittel aus 4,0 Gew.-% Vanadium (berechnet als V₂O₅), 0,40 Gew.-% Cäsium (berechnet als Cs) und 95,6 Gew.-% Titandioxid.

### B2 Herstellung einer V₂O₅/TiO₂-Phase mit 0,5 Gew.-% Cs

In 100 ml vollentsalztes Wasser von 60 °C gab man unter Rühren 13,0 g Oxalsäuredihydrat (= 0,12 Mol) und dann 3,35 g V₂O₅ (= 0,17 Mol). Die erhaltene blaue Lösung wurde 30 min unter weiterem Rühren auf 90 °C erhitzt. Nach dem Abkühlen auf Raumtemperatur fügte man 0,58 g Cäsiumsulfat (= 0,0016 Mol), 38,9 g Formamid, 80 g Anatas mit einer BET-Oberfläche von 21 m²/g und 157,4 g Wasser hinzu (Feststoffgehalt etwa 21 %). Anschließend rührte man die erhaltene Suspension 15 Stunden bei 25 °C. Dann wurde die Suspension sprühgetrocknet (Eingangstemperatur (Luft) = 350 °C; Ausgangstemperatur (Luft) = 110 °C). Das Sprühpulver bestand im Mittel aus 4,0 Gew.-% Vanadium (berechnet als V₂O₅), 0,50 Gew.-% Cäsium (berechnet als Cs) und 95,6 Gew.-% Titandioxid.

### Katalysatorherstellung

### Aufbringung der Phasen als Pulvermischung auf Kugeln

Zur Herstellung der Katalysatoren 1 bis 7 wurde das Pulver A mit 0 bis 20 Gew.% Pulver B1 bzw. B2 gemischt (siehe Tabelle) und wie folgt auf Magnesiumsilikat-Kugeln aufgebracht: 300 g Steatit-Kugeln mit einem Durchmesser von 3,5 bis 4 mm wurden in einer Dragiertrommel bei 20 °C während 20 min mit 40 g des gemischten Pulvers und 4,4 g Oxalsäure unter Zusatz von 35,3 g eines 60 Gew.-% Wasser und 40 Gew.-% Glycerin enthaltenden Gemisches beschichtet und anschließend getrocknet. Das Gewicht der so aufgetragenen katalytisch aktiven Masse, bestimmt an einer Probe des erhaltenen Präkatalysators, betrug nach einstündiger Wärmebehandlung bei 400 °C 10 Gew.-%, bezogen auf das Gesamtgewicht des fertigen Katalysators.

### Aufbringung der Phasen in Form konzentrischer Schalen auf Kugeln

Zur Herstellung des Katalysators 8 wurden die Pulver A und B1 wie folgt auf Magnesiumsilikat-Kugeln aufgebracht: 300 g Steatit-Kugeln mit einem Durchmesser von 3,5 bis 4 mm wurden in einer Dragiertrommel bei 20 °C während 20 min mit 36 g des Pulvers A und 4 g Oxalsäure beschichtet. Die beschichteten Kugeln wurden dann mit 6 g des Pulvers B1 unter Zusatz von 30 g eines 60 Gew.-% Wasser und 40 Gew.-% Glycerin enthaltenden Gemisches beschichtet und anschließend getrocknet. Das Gewicht der so aufgetragenen katalytisch aktiven Masse, bestimmt an einer Probe des erhaltenen Präkatalysators, betrug nach einstündiger Wärmebehandlung bei 400 °C 10 Gew.-%, bezogen auf das Gesamtgewicht des fertigen Katalysators, wobei 86 Gew.-% auf die innere Schicht der Silber-Vanadiumoxid-Bronze und 14 Gew.-% auf die äußere Schicht der V₂O₅/TiO₂-Phase entfielen.

### Aufbringung der Phasen als Pulvermischung auf Ringe

Für die Anwendungsbeispiele 2 bis 4 wurde das Pulver A mit 0 bzw. 10 Gew. Pulver B1 gemischt und wie folgt auf Magnesiumsilikat-Ringe aufgebracht: 350 g Steatit-Ringe mit einem äußeren Durchmesser von 7 mm, einer Länge von 3 mm und einer Wandstärke von 1,5 mm wurden in einer Dragiertrommel bei 20 °C während 20 min mit 84,4 g des gemischten Pulvers und 9,4 g Oxalsäure unter Zusatz von 66,7 g eines 60 Gew.-% Wasser und 40 Gew.-% Glycerin enthaltenden Gemisches beschichtet und anschließend getrocknet. Das Gewicht der so aufgetragenen katalytisch aktiven Masse, bestimmt an einer Probe des erhaltenen Präkatalysators, betrug nach einstündiger Wärmebehandlung bei 450 °C 18 Gew.-%, bezogen auf das Gesamtgewicht des fertigen Katalysators.

### Referenzkatalysator 1 (V₂O₅/TiO₂)

1400 g Steatit (Magnesiumsilikat) Ringe mit einem äußeren Durchmesser von 8 mm, einer Länge von 6 mm und einer Wandstärke von 1,6 mm wurden in einer Dragiertrommel auf 160 °C erhitzt und zusammen mit 13,8 g eines organischen Binders, bestehend aus einem Copolymer von Acrylsäure/Maleinsäure (Gewichtsverhältnis 75:25), mit einer Suspension aus 466 g Anatas mit einer BET-Oberfläche von 21 m²/g, 67,2 g Vanadyloxalat, 14,4 g Antimontrioxid, 3,15 g Ammoniumhydrogenphosphat, 2,87 g Cäsiumsulfat, 721 g Wasser und 149 g Formamid besprüht. Die auf diese Weise aufgebrachte katalytisch aktive Masse bestand im Mittel aus 0,16 Gew.-% Phosphor (berechnet als P), 7,5 Gew.-% Vanadium (berechnet als V₂O₅), 3,2 Gew.-% Antimon (berechnet als Sb₂O₃), 0,40 Gew.-% Cäsium (berechnet als Cs) und 88,74 Gew.-% Titandioxid.

Der so erhaltene Schalenkatalysator wurde in einer Dragiertrommel auf 160 C erhitzt und zusammen mit 14 g eines organischen Binders, bestehend aus einem Copolymer von Acrylsäure/Maleinsäure (Gewichtsverhältnis 75:25), mit einer Suspension aus 502 g Anatas mit einer BET-Oberfläche von 21 m²/g, 35,8 g Vanadyloxalat, 2,87 g Cäsiumsulfat, 720 g Wasser und 198 g Formamid besprüht. Die auf diese Weise aufgebrachte katalytisch aktive Masse bestand im Mittel aus 4,0 Gew.-% Vanadium (berechnet als V₂O₅), 0,4 Gew.-% Cäsium (berechnet als Cs) und 88,8 Gew.-% Titandioxid. Das Gewicht der aufgetragenen Schichten betrug 9,3 Gew.-% des Gesamtgewichts des fertigen Katalysators.

### Referenzkatalysator 2 (V₂O₅/TiO₂-Katalysator)

1400 g Steatit (Magnesiumsilikat) Ringe mit einem äußeren Durchmesser von 8 mm, einer Länge von 6 mm und einer Wandstärke von 1,6 mm wurden in einer Dragiertrommel auf 160 °C erhitzt und mit einer Suspension aus 468 g Anatas mit einer BET-Oberfläche von 21 m²/g, 67,2 g Vanadyloxalat, 16,8 g Antimontrioxid, 2,95 g Ammoniumhydrogenphosphat, 0,72 g Cäsiumsulfat, 719 g Wasser und 150 g Formamid besprüht, bis das Gewicht der auf getragenen Schicht 10,5 % des Gesamtgewichts des fertigen Katalysators betrug (nach einstündiger Wärmebehandlung bei 450 °C). Die auf diese Weise aufgebrachte katalytisch aktive Masse, also die Katalysatorschale, bestand im Mittel aus 0,15 Gew.-% Phosphor (berechnet als P), 7,5 Gew.-% Vanadium (berechnet als V₂O₅), 3,2 Gew.-% Antimon (berechnet als Sb₂O₃), 0,1 Gew.-% Cäsium (berechnet als Cs) und 89,05 Gew.-% Titandioxid.

### Anwendungsbeispiele

### 1. Herstellung von Phthalsäureanhydrid

In ein 80 cm langes Eisenrohr mit einer lichten Weite von 16 mm wurden die Katalysatoren gemäß der Tabelle (beschichtete Steatit-Kugeln) bis zu einer Bettlänge von 66 cm eingefüllt. Das Eisenrohr war zur Temperaturregelung mit einem Elektroheizmantel umgeben. Bei den Versuchen beträgt die Temperatur 350°C. Durch das Rohr wurden stündlich von oben nach unten 360 NI-Luft mit einer Beladung an 98,5 gew.%igem o-Xylol von 60 g o-Xylol/Nm³ Luft geleitet. In der nachstehenden Tabelle sind die erhaltenen Ergebnisse zusammengefasst.

**Tabelle**

| Katalysator | Ag_{0,73}V₂Oₓ-Phase A | V₂O₅/TiO₂-Phase | | Umsatz (Mol-%) | COₓ-Selektivität¹⁾ (Mol-%) |
|---|---|---|---|---|---|
| | | B1 | B2 | | |
| 1 | - | 100% | - | 81 | 33 |
| 2 | 100% | - | - | 37 | 10 |
| 3 | 98% | 2% | - | 49 | 16 |
| 4 | 95% | 5% | - | 44 | 11 |
| 5 | 90% | 10% | - | 42 | 12 |
| 6 | 80% | 20% | - | 35 | 12 |
| 7 | 90% | - | 10% | 44 | 9 |
| 8 | 86%²⁾ | 14%²⁾ | - | 49 | 9 |

| | | | | | |
|---|---|---|---|---|---|
| ¹⁾ "COₓ-Selektivität" entspricht dem Anteil des zu Verbrennungsprodukten (CO, CO₂) umgesetzten o-Xylols; die Restselektivität auf 100 % entspricht dem Anteil des zu dem Wertprodukt Phthalsäureanhydrid und den Zwischenprodukten o-Tolylaldehyd, o-Tolylsäure und Phthalid sowie Nebenprodukten wie Maleinsäureanhydrid, Citraconsäureanhydrid und Benzoesäure umgesetzten o-Xylols ²⁾ konzentrisch aufgebrachte Schalen | | | | | |

An einer Ausbauprobe des Katalysators 2 (mit 100% Phase A) wurde eine BET-Oberfläche der Aktivmasse von 6,7 m²/g und eine Vanadium-Oxidationsstufe von 4,63 ermittelt. Aus dem Pulverröntgendiagramm wurden die folgenden Netzebenenabstände d [Å] mit den dazugehörigen relativen Intensitäten Iᵣₑₗ [%] erhalten: 4,85 (9,8), 3,50 (14,8), 3,25 (39,9), 2,93 (100), 2,78 (36,2), 2,55 (35,3), 2,43 (18,6), 1,97 (15,2), 1,95 (28,1), 1,86 (16,5), 1,83 (37,5), 1,52 (23,5). Die Ausbauproben der Katalysatoren 3-7 zeigten keine Änderungen der Ag/V-Phase hinsichtlich Pulverröntgendiagramm, BET-Oberfläche (etwa 6,7 m²/g) und Vanadium-Oxidationsstufe (4,67).

### 2. Herstellung von Phthalsäureanhydrid (Vergleichsbeispiel)

Von unten nach oben wurden jeweils 0,80 m des Referenzkatalysators 2, 1,40 m des Referenzkatalysators 1 und anschließend 0,80 m eines Katalysators (beschichtete Steatit-Ringe), dessen Aktivmasse aus 100 Gew.-% A bestand, in ein 3,85 m langes Eisenrohr mit einer lichten Weite von 25 mm eingefüllt. Das Eisenrohr war zur Temperaturregelung von einer Salzschmelze umgeben. Durch das Rohr wurden stündlich von oben nach unten 4,0 Nm³ Luft mit einer Beladung an 98,5 gew.-%igem o-Xylol von 80 g o-Xylol/Nm³ Luft geleitet. Dabei wurde bei einer Salzbadtemperatur von 353 bis 360°C eine durchschnittliche PSA-Ausbeute von 115,5 Gew.-% erreicht (Ausbeute bedeutet das erhaltene Phthalsäureanhydrid in Gewichtsprozent, bezogen auf 100%iges o-Xylol). Der Umsatz betrug mehr als 99,94 %, der Rest-Phthalid-Gehalt am Reaktorausgang lag bei weniger als 0,35 Gew.-%.

### 3. Herstellung von Phthalsäureanhydrid

Anwendungsbeispiel 2 wurde wiederholt, wobei jedoch jeweils 1,00 m des Referenzkatalysators 2, 1,60 m des Referenzkatalysators 1 und anschließend 0,40 m eines Katalysators (beschichtete Steatit-Ringe), dessen Aktivmasse aus 90 Gew.-% A und 10 Gew.-% B1 bestand, eingefüllt wurden. Es wurde eine durchschnittliche PSA-Ausbeute von 116,4 Gew.-% erreicht. Der Umsatz betrug mehr als 99,94 %, der Rest-Phthalid-gehalt am Reaktorausgang lag bei weniger als 0,30 Gew.-%. Dieses Beispiel zeigt, dass bei Verwendung der erfindungsgemäßen Katalysatoren hohe PSA-Ausbeuten auch mit einer gegenüber dem Anwendungsbeispiel 2 deutlich verkürzten Bettlänge des Silber-Vanadiumoxid-Katalysators erreicht werden können.

### 4. Herstellung von Phthalsäureanhydrid

Anwendungsbeispiel 2 wurde wiederholt, wobei jedoch jeweils 0,80 m des Referenzkatalysators 2, 1,80 m des Referenzkatalysators 1 und anschließend 0,40 m eines Katalysators (beschichtete Steatit-Ringe), dessen Aktivmasse aus 90 Gew.-% A und 10 Gew.-% B1 bestand, eingefüllt wurden. Es wurde eine durchschnittliche PSA-Ausbeute von 116,9 Gew.-% erreicht. Der Umsatz betrug mehr als 99,94 %, der Rest-Phthalid-Gehalt am Reaktorausgang lag bei weniger als 0,35 Gew.-%. Dieses Beispiel zeigt, dass bei Verwendung der erfindungsgemäßen Katalysatoren hohe PSA-Ausbeuten mit einer gegenüber dem Anwendungsbeispiel 2 angepassten Schüttungslängenverteilung der Referenzkatalysatoren erreicht werden können.

## Patentansprüche

1. Katalysator, umfassend eine auf einen inerten Träger aufgebrachte katalytisch aktive Masse, die eine Phase A und eine Phase B in Form dreidimensional ausgedehnter, von ihrer lokalen Umgebung aufgrund ihrer von ihrer lokalen Umgebung verschiedenen chemischen Zusammensetzung abgegrenzter Bereiche enthält, wobei die Phase A eine Silber-Vanadiumoxid-Bronze und die Phase B eine Mischoxidphase auf Basis von Titandioxid und Vanadiumpentoxid ist, wobei die Phasen A und B relativ zueinander wie in einem Gemisch aus feinteiligem A und feinteiligem B verteilt sind oder die Phasen A und B relativ zueinander als konzentrische Schalen angeordnet sind.

2. Katalysator nach Anspruch 1, wobei das Gewichtsverhältnis der Phase A zur Phase B im Bereich von 85:15 bis 95:5 liegt.

3. Katalysator nach einem der vorhergehenden Ansprüche, wobei die Phase A eine Zusammensetzung aufweist, die erhältlich ist durch Calzinieren eines Multimetalloxids der allgemeinen Formel I
Ag_{a-c}M¹_{c}V₂O_{d} * e H₂O, I
worin
a einen Wert von 0,3 bis 1,9 hat,
M¹ für wenigstens ein unter Alkali- und Erdalkalimetallen, Bi, Tl, Cu, Zn, Cd, Pb, Cr, Au, Al, Fe, Co, Ni, Mo, Nb, Ce, W, Mn, Ta, Pd, Pt, Ru und/oder Rh ist ausgewähltes Metall steht,
c einen Wert von 0 bis 0,5 hat, mit der Maßgabe, dass (a-c) ≥ 0,1 ist,
d eine Zahl, die sich durch die Wertigkeit und Häufigkeit der von Sauerstoff verschiedenen Elemente in der Formel I bestimmt, bedeutet und
e einen Wert von 0 bis 20 hat.

4. Katalysator nach einem der vorhergehenden Ansprüche, wobei die Phase B eine Zusammensetzung der Formel aufweiset
VₓTi₁₋ₓM²_{y}M³_{w}O_{z} II
worin
M² für wenigstens ein Alkalimetall steht;
M³ für ein Element der 5. Hauptgruppe des Periodensystems der Elemente steht,
x einen Wert von 0,001 bis 0,2 hat,
y einen Wert von 0 bis 0,01 hat,
w einen Wert von 0 bis 0,02 hat, und
z eine Zahl, die sich durch die Wertigkeit und Häufigkeit der von Sauerstoff verschiedenen Elemente in der Formel II bestimmt, bedeutet.

5. Verfahren zur Herstellung eines Katalysators nach Anspruch 1, bei dem man ein Pulver, das die Phase A, einen Vorläufer dafür oder Quellen der elementaren Konstituenten davon umfasst, und ein Pulver, das die Phase B, einen Vorläufer dafür oder Quellen der elementaren Konstituenten davon umfasst, vermengt und auf einen inerten Träger aufbringt.

6. Verfahren zur Herstellung eines Katalysators nach Anspruch 1, bei dem man nacheinander (i) die Phase A, einen Vorläufer dafür oder Quellen der elementaren Konstituenten davon und (ii) die Phase B, einen Vorläufer dafür oder Quellen der elementaren Konstituenten davon auf einen inerten Träger aufbringt.

7. Verfahren zur Herstellung von Aldehyden, Carbonsäuren und/oder Carbonsäureanhydriden, bei dem man einen gasförmigen Strom, der einen aromatischen oder heteroaromatischen Kohlenwasserstoff und ein molekularen Sauerstoff enthaltendes Gas umfasst, bei erhöhter Temperatur mit einem Katalysator nach einem der Ansprüche 1 bis 4 in Kontakt bringt.

8. Verfahren nach Anspruch 7, bei dem man den gasförmigen Strom nacheinander über ein Bett eines stromaufwärts gelegenen Katalysators und ein Bett eines stromabwärts gelegenen Katalysators leitet, wobei das Bett des stromaufwärts gelegenen Katalysators einen Katalysator nach einem der Ansprüche 1 bis 7 enthält und das Bett des stromabwärts gelegenen Katalysators wenigstens einen Katalysator enthält, dessen katalytisch aktive Masse aus einer Mischoxidphase auf Basis von Titandioxid und Vanadiumpentoxid besteht.

9. Verfahren nach Anspruch 7 oder 8, bei dem man als aromatischen Kohlenwasserstoff o-Xylol oder Naphthalin oder Mischungen aus o-Xylol und Naphthalin zu Phthalsäureanhydrid oxidiert.

## Claims

1. A catalyst comprising a catalytically active composition which has been applied to an inert support comprises a phase A and a phase B in the form of three-dimensional regions delimited from their local environment owing to their different chemical composition from their local environment, wherein phase A is a silver-vanadium oxide bronze and phase B a mixed oxide phase based on titanium dioxide and vanadium pentoxide and, wherein phases A and B are distributed relative to one another as in a mixture of finely divided A and finely divided B or phases A and B are arranged relative to one another as concentric shells.

2. The catalyst according to claim 1, wherein the weight ratio of phase A to phase B is in the range from 85:15 to 95:5.

3. The catalyst according to either of the preceding claims, wherein phase A has a composition which is obtainable by calcining a multimetal oxide of the general formula I
Ag_{a-c}M¹ _{c}V₂O_{d} * e H I
where
a has a value from 0.3 to 1.9,
M¹ is at least one metal selected from alkali metals and alkaline earth metals, Bi, Tl, Cu, Zn, Cd, Pb, Cr, Au, Al, Fe, Co, Ni, Mo, Nb, Ce, W, Mn, Ta, Pd, Pt, Ru and/or Rh,
c has a value from 0 to 0.5, with the proviso that (a-c) ≥ 0.1,
d is a number which is determined by the valency and frequency of the elements in the formula I other than oxygen, and
e has a value from 0 to 20.

4. The catalyst according to any of the preceding claims, wherein phase B has a composition of the formula II
VₓTi₁₋ₓM²_{y}M³_{w}O_{z} II
where
M² is at least one alkali metal;
M³ is an element of main group 5 of the Periodic Table of the Elements,
x has a value from 0.001 to 0.2,
y has a value from 0 to 0.01,
w has a value from 0 to 0.02, and
z is a number which is determined by the valency and frequency of the elements in the formula II other than oxygen.

5. A process for preparing the catalyst according to claim 1, in which a powder which comprises phase A, a precursor therefor or sources of the elemental constituents thereof, and a powder B which comprises phase B, a precursor therefor or sources of the elemental constituents thereof are mixed and applied to an inert support.

6. A process for preparing the catalyst according to claim 1, in which (i) phase A, a precursor therefor or sources of the elemental constituents thereof and (ii) phase B, a precursor therefor or sources of the elemental constituents thereof are applied successively to an inert support.

7. A process for preparing aldehydes, carboxylic acids and/or carboxylic anhydrides, in which a gaseous stream which comprises an aromatic or heteroaromatic hydrocarbon and a molecular oxygen-comprising gas are contacted at elevated temperature with the catalyst according to any of claims 1 to 4.

8. The process according to claim 7, in which the gaseous stream is passed successively over a bed of a catalyst disposed upstream and a bed of a catalyst disposed downstream, the bed of the catalyst disposed upstream comprising the catalyst according to any of claims 1 to 7 and the bed of the catalyst disposed downstream comprising at least one catalyst whose catalytically active composition consists of a mixed oxide phase based on titanium dioxide and vanadium pentoxide.

9. The process according to claim 7 or 8, in which the aromatic hydrocarbon oxidized is o-xylene or naphthalene or a mixture of o-xylene and naphthalene to give phthalic anhydride.

## Revendications

1. Catalyseur, comprenant appliquée sur un support inerte une masse catalytiquement active qui contient une phase A et une phase B sous forme de zones à extension tridimensionnelle, délimitées par rapport à leur environnement local en raison de leur composition chimique différente de celle de leur environnement local, la phase A étant un bronze d'oxyde de vanadium-argent et la phase B étant une phase d'oxyde mixte à base de dioxyde de titane et pentoxyde de vanadium, les phases A et B étant réparties l'une par rapport à l'autre comme dans un mélange de A finement divisée et B finement divisée ou les phases A et B étant disposées l'une par rapport à l'autre sous forme de couches concentriques.

2. Catalyseur selon la revendication 1, dans lequel le rapport pondéral de la phase A à la phase B se situe dans la plage de 85:15 à 95:5.

3. Catalyseur selon l'une quelconque des revendications précédentes, dans lequel la phase A présente une composition qui peut être obtenue par calcination d'un oxyde multimétallique de formule générale I
Ag_{a-c}M¹cV₂O_{d} * e H₂O I
dans laquelle
a a une valeur de 0,3 à 1,9,
M¹ représente au moins un métal choisi parmi les métaux alcalins et alcalino-terreux, Bi, Tl, Cu, Zn, Cd, Pb, Cr, Au, Al, Fe, Co, Ni, Mo, Nb, Ce, W, Mn, Ta, Pd, Pt, Ru et/ou Rh,
c est une valeur de 0 à 0,5, étant entendu que (a-c) ≥ 0,1,
d représente un nombre qui est dicté par la valence et la fréquence des éléments différents de l'oxygène dans la formule I, et
e a une valeur de 0 à 20.

4. Catalyseur selon l'une quelconque des revendications précédentes, dans lequel la phase B présente une composition de formule II
VₓTi₁₋ₓM²_{y}M³_{w}O_{z} II
dans laquelle
M² représente au moins un métal alcalin ;
M³ représente un élément du groupe 5A du système périodique des éléments,
x a une valeur de 0,001 à 0,2,
y a une valeur de 0 à 0,01,
w a une valeur de 0 à 0,02, et
z représente un nombre qui est dicté par la valence et la fréquence des éléments différents de l'oxygène dans la formule II.

5. Procédé pour la préparation d'un catalyseur selon la revendication 1, dans lequel on mélange et applique sur un support inerte une poudre qui comprend la phase A, un précurseur de celle-ci ou des sources des constituants élémentaires de celle-ci et une poudre qui comprend la phase B, un précurseur de celle-ci ou des sources des constituants élémentaires de celle-ci.

6. Procédé pour la préparation d'un catalyseur selon la revendication 1, dans lequel on applique sur un support inerte successivement (i) la phase A, un précurseur de celle-ci ou des sources des constituants élémentaires de celle-ci et (ii) la phase B, un précurseur de celle-ci ou des sources des constituants élémentaires de celle-ci.

7. Procédé pour la production d'aldéhydes, d'acides carboxyliques et/ou d'anhydrides d'acides carboxyliques, dans lequel on met en contact un courant gazeux, qui comprend un hydrocarbure aromatique ou hétéroaromatique, et un gaz contenant de l'oxygène moléculaire, à température élevée, avec un catalyseur selon l'une quelconque des revendications 1 à 4.

8. Procédé selon la revendication 7, dans lequel on fait passer le courant gazeux successivement sur un lit d'un catalyseur placé en amont et un lit d'un catalyseur placé en aval, le lit du catalyseur placé en amont contenant un catalyseur selon l'une quelconque des revendications 1 à 7 et le lit du catalyseur placé en aval contenant au moins un catalyseur dont la masse catalytiquement active consiste en une phase d'oxyde mixte à base de dioxyde de titane et pentoxyde de vanadium.

9. Procédé selon la revendication 7 ou 8, dans lequel on oxyde en tant qu'hydrocarbure aromatique de l'o-xylène et du naphtalène ou des mélanges d'o-xylène et de naphtalène en anhydride phtalique.
